# EUROPEAN PATENT SPECIFICATION

(11) **EP 1 240 895 B1**
(45) Date of publication and mention of the grant of the patent: **21.06.2006**
(21) Application number: 02076010.4
(22) Date of filing: 12.03.2002
(51) Int. Cl.: A61Q 19/00, A61K 8/44

(54) **Use of alkyl 3-(N-alkylacetamino)propionate derivatives as moisturizing agents**
Verwendung von Alkyl 3-(N-alkylacetamino)propionat Derivaten als Feuchthaltemittel
Utilisation de l'alkyl 3-(N-alkylacetamino)propionate comme agent hydratant

(30) Priority: 14.03.2001 EP 01200953
(43) Date of publication of application: 18.09.2002
(73) Proprietor: JOHNSON & JOHNSON GmbH, 40474 Düsseldorf (DE)
(72) Inventor: Von Setten, Otto, 52072 Aachen (DE); Schulte, Petra, 41466 Neuss (DE)
(74) Representative: Weber-Bruls, Dorothée

(56) References cited:
- EP-A- 0 962 135
- DE-A- 2 234 399
- US-A- 5 230 890
- US-A- 5 882 633
- PATENT ABSTRACTS OF JAPAN vol. 2000, no. 15, 6 April 2001 (2001-04-06) & JP 2000 355516 A (AJINOMOTO CO INC), 26 December 2000 (2000-12-26)

## Description

### Brief description of the invention

This invention concerns the use of ethyl 3-(N-butylacetamino)propionate as moisturizer.

### Background of the invention

The outer part of the skin or stratum corneum contains a certain quantity of water that acts as a kind of plasticizer keeping it smooth and supple and furthermore reassures sufficient skin permeability. Keeping the proper water content in the outer part of the skin is essential for keeping its integrity and avoiding adverse effects related to insufficient water content. An important role herein is played by the presence of skin lipids that are believed to act as a natural water barrier to maintain the skin's water content at a sufficient level.

There are several factors that can negatively influence the water balance in the outer part of the skin. If the ambient humidity is too low, such as in a cold climate, insufficient water remains in the stratum corneum and the skin begins to scale and becomes itchy.

Another factor that causes a disturbed water balance relates to our daily hygiene in which the skin is subjected to frequent washing and cleansing. Personal care products used for that purpose contain ingredients such as anionic surfactants and organic solvents that penetrate the stratum corneum and, by removal of the lipids from that part of the skin, affect its integrity. This leads to the adverse effects generally associated with a dry skin such as rough feel, itching, irritation and the like.

A number of people have a natural tendency to have a dry skin without any external causes or which are very sensitive to personal hygiene products. This may be caused, for example, by a deficiency of skin lipid components.

To deal with these problems, compositions have been developed which assist the stratum corneum in maintaining its barrier and water-retention functions at optimum performance in spite of deleterious interactions which the skin may encounter in washing, work, and recreation. These compositions typically contain moisturizing agents which are lipid in nature and come in a wide variety of forms such as creams, lotions and with varying degrees of emolliency, barrier and water-retention (moisturizing) benefits.

However there is a need for other skin-care compositions which provide excellent moisturization, absorption, skin feel, skin care and appearance characteristics and which in particular provide improved short and longer term moisturizing effectiveness. These compositions should also display reduced stickiness and avoid a greasy feel on the skin while also having excellent stability characteristics at both normal and elevated temperatures.

The compound ethyl 3-(N-butylacetamino)propionate is a well-known insect repellant which is commercially available. Insect repellant compositions that contain this compound and close analogs thereof have been disclosed in EP-A-0 962 135. This compound has further been described as an agent that improves efficacy of sunscreen agents in US-5,882,633.

DE 2 234 399 OS is about a skin care agent of the formula R¹-C(O)-N(R²)-C(R³)(H)-(CH₂)ₙ-COOR⁴ which shall have therapeutic and cosmetic properties suitable for the treatment and care of the skin. In this formula R¹ is H or C₁₋₂₄ alkyl, R² is H or C₁₋₃ alkyl, R³ is H or C₁₋₆ alkyl, and R⁴ is C₁₋₂₄ alkyl. Among others, these compounds shall have an inhibitory effect on sebaceous glands, and shall be anti-phlogistic, anti-prolific, anaesthetic, skin caring, skin protecting, and shall increase the moisture content of the skin, stabilize capillaries and be useful against dandruff. DE 2 234 399 OS acknowledges that a plethora of ethylamino carbonic acid derivatives has been described without knowing their biological effects.

Quite unexpectedly it has been found that this compound and its analogs have excellent moisturizing properties and therefore can be used as a moisturizer in cosmetic and topical pharmaceutical compositions. Furthermore it has been found that it is possible to provide compositions having the above mentioned advantageous properties.

### Summary of the Invention

The present invention concerns the cosmetic use of ethyl 3-(N-butylacetamino)propionate as a skin moisturizer. More in particular it concerns the use of this compound as a moisturizing agent in cosmetic formulations.

In a further aspect there is provided a cosmetic method of moisturizing the skin, said method comprising applying to the skin an effective amount of ethyl 3-(N-butylacetamino)propionate, preferably in a suitable cosmetic composition.

In a further aspect there is provided a cosmetic method of treating a subject suffering from dry skin, said method comprising topically administering an effective amount of ethyl 3-(N-butylacetamino)propionate, preferably in a suitable cosmetic composition.

In still a further aspect there is provided ethyl 3-(N-butylacetamino)propionate for use as a medicine. Furthermore there is provided ethyl 3-(N-butylacetamino)propionate for use as a moisturizing medicine, or for use as a medicine for moisturizing the skin or alternatively for treating dry skin, or for treating skin conditions associated with dry skin.

Or in a further aspect there is provided the use of ethyl 3-(N-butylacetamino)propionate for the manufacture of a topical pharmaceutical formulation for moisturizerizing the skin, or alternatively for treating dry skin, or for treating skin conditions associated with dry skin.

### Detailed Description of the Invention

As used herein "C₁₋₆alkyl" defines straight and branched chain hydrocarbon radicals having from 1 to 6 carbon atoms such as, for example, methyl, ethyl, propyl, 1-methylethyl, butyl, 1-methylpropyl, 2-methylpropyl, pentyl, 1-methylbutyl, 2-methylbutyl, hexyl, 1-methylpentyl, 2-methylpentyl, and the like.

Ethyl 3-(N-butylacetamino)propionate, which is also named ethyl N-butyl-N-acetyl aminopropionate, can be represented by the formula:

CH₃-CO-N(n.C₄H₉)-CH₂CH₂-COOC₂H₅ (I-a)

This compound is commercially available and is used as an insect repellant.

It now has been found that the compound (I-a) has moisturizing properties and therefore can be used as moisturizing agent.

In order to act as a moisturizer in cosmetical applications, the compound of formula (I-a) is preferably formulated into appropriate cosmetic compositions appropriate, e.g. any of the art-known formulations aimed at moisturizing the skin. In particular, the moisturizing cosmetic compositions can be in the form of creams, ointments, lotions, gels, solutions, hydrophilic lotions, and the like. When oily components are present, he formulations may be formulated as emulsions such as, for example, oil-in-water, water-in-oil or multiple emulsions such as water-in-oil-in-water, or in liposome form. The compositions may also be lotions for application on the skin by means of wipes.

Or the compound of formula (I-a) may be used as an additive in cosmetic products in which a moisturizing effect is desirable. Compositions to which the addition of a moisturizing agent is desirable are those containing agents that may have a drying effect on the skin such as, for example, surfactants, in particular non-ionic surfactants and/or organic solvents. Examples comprise cleansing compositions such as soaps, shampoos, cleansing lotions and the like.

The cosmetic compositions containing the compound of formula (I-a) may additionally contain further ingredients or additives typically used in such compositions such as surfactants, emulsifiers, consistency factors, conditioners, emollients, skin caring ingredients, other moisturizers, thickeners, lubricants, fillers, anti-oxidants, preservatives, active ingredients, fragrances and the like. Active ingredients as mentioned herein comprise all active ingredients suited for topical applications such as, for example, anti-inflammatories, anti-bacterials, anti-fungals and the like agents.

The invention further concerns the use as a medicine or the use for the manufacture of medicament of ethyl 3-(N-butylacetamino)propionate. To this purpose the moisturizing agent of formula (I-a) is formulated into a suitable pharmaceutical composition for topical application, which contains an effective amount of ethyl 3-(N-butylacetamino)propionate, and a suitable carrier. Suitable carriers comprise components typically used in topical pharmaceutical formulations such as for example surfactants, emulsifiers, solvents in particular solvents to assist penetration of the active ingredient, consistency factors, emollients, thickeners, lubricants, fillers, anti-oxidants, preservatives, other active ingredients, fragrances and the like. Suitable topical pharmaceutical formulations may be presented as, for instance, ointments, jellies, creams, lotions, impregnated dressings. The carriers may be present in concentrations in the range from about 1% up to about 98% of the formulation, in particular up to about 80% of the formulation (all % being w/w).

### Examples

### Example 1

### Skin Moisturisation

### Test Method

For the determination of skin moisture Corneometry using a Corneometer CM 820 PC (Courage and Khazaka, Cologne, Germany) was applied. The instrument registers the electrical capacitance of the skin surface in arbitrary units. The probe head (7x7 mm) consisting of a condenser was applied to the skin surface at constant pressure (3,5 N). The measurement principle is based on distinctly different dielectric constants of water and most other materials. Three measurements were performed on each test area and the mean was used to define the hydration state of the stratum corneum.

### Study design

2,2 mg/cm² +/- 0,02 of the product (Ethyl Butylacetylaminopropionate) were applied to the inner forearm of human volunteers. The product was spread evenly over the test area using a presaturated-gloved finger. The actual amount on the skin was determined by the weighing per loss method. One field remained untreated and served as control area.

Skin moisture was measured pre-test, at the start, 0.5, 1, 2, 4, and 8 hours after application.

| Increase of Skin Moisture (%) | | | | | | |
|---|---|---|---|---|---|---|
| Time (h) | 0 | 0,5 | 1 | 2 | 4 | 8 |
| untreated | 0 | 1 | 1,6 | -7 | 2,2 | -2,2 |
| product | 0 | 17,9 | 28 | 31,3 | 22,4 | 5,6 |

The product led to an significant increase in skin moisturisation up to 8 h after one single application compared to the untreated area and the pre-test values.

### Example 2 (does not fall within the claims)

**Moisturizing cream**

| | |
|---|---|
| Component | % (w/w) |
| Aqua | 57.75 |
| Acrylates/C10-30 alkyl acrylate cross polymer | 0.75 |
| Disodium EDTA | 0.10 |
| Methyl Paraben (20%) | 1.75 |
| Propyl Paraben (7%) | |
| Phenoxyethanol (73%) | |
| Glyceryl polymethacrylate 67% | 10.75 |
| Aqua 32% | |
| Propylene glycol 1% | |
| Butylene glycol | 6.00 |
| Sodium hydroxyde | 0.04 |
| Cetearyl alcolhol | 1.75 |
| C12-C15 alkyl benzoate | 6.00 |
| Potassium cetyl phosphate | 3.00 |
| Vitamin C | 0.01 |
| Tocopheryl acetate | 1.00 |
| Talc | 1.10 |
| Glycerin (100%) | 6.25 |
| Ginkgo biloba <92.2% | 0.05 |
| Ethanol <3% | |
| Aqua <3% | |
| Perfume | 0.20 |
| Cyclomethicone | 3.50 |

Starting with water, the above formulation was prepared by adding, under stirring, the ingredients in the sequence they are listed.

## Claims

1. Cosmetic use as a skin moisturizer of ethyl 3-(N-butylacetamino)propionate in cosmetic formulations.

2. Use of ethyl-3-(N-butylacetamino)propionate for the manufacture of a medicament for treating dry skin.

## Patentansprüche

1. Kosmetische Verwendung von Ethyl-3-(N-butylacetamino)propionat als ein Hautfeuchthaltemittel in kosmetischen Formulierungen.

2. Verwendung von Ethyl-3-(N-butylacetamino)propionat zur Herstellung eines Medikaments zur Behandlung trockener Haut.

## Revendications

1. Utilisation cosmétique en tant qu'hydratant pour la peau du 3-(N-butylacétamino)propionate d'éthyle dans des formulations cosmétiques.

2. Utilisation du 3-(N-butylacétamino)propionate d'éthyle pour la fabrication d'un médicament pour le traitement des peaux sèches.
